# FASCICULE DE BREVET EUROPEEN

(11) **EP 2 961 450 B1**
(45) Date de publication et mention de la délivrance du brevet: **01.11.2017**
(21) Numéro de dépôt: 14713172.6
(22) Date de dépôt: 25.02.2014
(51) Int. Cl.: A61M 5/32, B29C 45/16, A61M 5/31

(54) **DISPOSITIF DE PROTECTION D'AIGUILLE**
NADELSCHUTZVORRICHTUNG
NEEDLE PROTECTION DEVICE

(30) Priorité: 01.03.2013 FR 1351841
(43) Date de publication de la demande: 06.01.2016
(73) Titulaire: Aptar Stelmi SAS, 93420 Villepinte (FR)
(72) Inventeur: FOURNIER, Arnaud, 75007 Paris (FR); FOURNIER, Ghislain, 17000 La Rochelle (FR); SWAL, Mickaël, 77124 Chauconin Neufmontiers (FR)
(74) Mandataire: CAPRI
(86) Numéro de dépôt international: PCT/FR2014/050389
(87) Numéro de publication internationale: WO 2014/131979

(56) Documents cités:
- WO-A1-2011/131996
- WO-A2-02/074367
- US-A- 5 084 027
- US-A1- 2009 143 746
- US-A1- 2009 187 153
- US-A1- 2009 198 196

## Description

La présente invention concerne un dispositif d'injection comportant un dispositif amovible de protection d'aiguille.

Les dispositifs de protection d'aiguille, aussi appelés protèges-aiguille, sont bien connus. Il en existe différents types, dont les protèges-aiguille rigides, comportant un corps interne en matériau souple et un corps externe en matériau rigide. Le corps interne assure l'étanchéité à la fois avec l'orifice de l'aiguille et avec le dispositif d'injection, en général le corps de seringue, alors que le corps externe sert à fixer et maintenir le protège-aiguille sur le dispositif d'injection jusqu'à l'utilisation de ce dernier. Les documents EP 1 208 861 et FR 2 777 787 décrivent notamment des dispositifs de protection d'aiguille de ce type.

Ces dispositifs peuvent présenter certains inconvénients. Ainsi, en raison notamment des tolérances de fabrication, en particulier des seringues en verre, il peut être difficile dans certains cas de garantir l'étanchéité en position de stockage entre le corps interne déformable du protège-aiguille et le dispositif d'injection, en l'occurrence le corps de seringue en verre. De plus, il n'est généralement pas possible d'empêcher un retrait non souhaité ou accidentel du protège-aiguille avant utilisation du dispositif d'injection associé, ce qui implique un risque de contamination de l'aiguille. De plus, la fabrication et l'assemblage du protège-aiguille, et notamment du corps externe sur le corps interne peut être complexe et donc coûteux.

Le document WO 2011/131996 décrit un dispositif de l'état de la technique avec un protège-aiguille en élastomère.

La présente invention a pour but de fournir un dispositif d'injection qui ne reproduit pas les inconvénients susmentionnés.

La présente invention a donc pour but de fournir un dispositif d'injection qui garantit l'étanchéité avant utilisation.

La présente invention a également pour but de fournir un dispositif d'injection qui soit simple et facile à fabriquer et à assembler, et fiable dans son utilisation.

La présente invention a donc pour objet un dispositif d'injection de produit fluide comportant un corps de seringue et une aiguille fixée dans une projection d'extrémité axiale du corps de seringue, ladite projection d'extrémité comportant une surface d'extrémité axiale, ledit dispositif d'injection comportant un dispositif de protection d'aiguille, ledit dispositif de protection étant, dans une position de stockage, fixé sur ledit dispositif d'injection, ledit dispositif de protection étant amovible dudit dispositif d'injection, ledit dispositif de protection comportant un corps interne en matériau sensiblement souple et/ou déformable et un corps externe en matériau sensiblement rigide, ledit corps interne, en position de stockage, obturant de manière étanche l'orifice de distribution de ladite aiguille et coopérant de manière étanche avec ledit dispositif d'injection, et ledit corps externe comportant une partie de fixation qui, en position de stockage, coopère avec un épaulement dudit dispositif d'injection pour fixer ledit dispositif de protection sur ledit dispositif d'injection, ledit corps interne comportant un bord d'extrémité axial proximal adapté, en position de stockage, à coopérer de manière étanche avec le corps de seringue uniquement avec ladite surface d'extrémité axiale, ledit corps interne étant réalisé en un matériau présentant les propriétés suivantes : une dureté supérieure à 55 Shore A, notamment supérieure à 60 Shore A, une élasticité supérieure à 8 MPa, notamment supérieure à 10 MPa, une déformation rémanente à la compression inférieure à 30%, notamment inférieure à 25%, une densité supérieure à 1, notamment supérieure à 1,3.

Avantageusement, ledit corps interne est réalisé en caoutchouc.

Avantageusement, ledit corps comporte une projection radiale et ledit corps externe comporte un épaulement radial et un bord d'extrémité radial définissant une ouverture axiale supérieure, ledit corps interne étant inséré dans ledit corps externe à travers ladite ouverture axiale supérieure, avec ladite projection radiale en butée sur ledit épaulement radial, ledit bord d'extrémité axial dudit corps externe étant rabattu sur ledit corps interne pour fixer ledit corps interne dans ledit corps externe.

Avantageusement, ladite partie de fixation est encliquetée sur ledit épaulement.

Avantageusement, ledit corps externe comporte une partie de protection fixée audit corps interne, ladite partie de protection reliée à ladite partie de fixation par au moins un pont de matière sécable, ledit au moins un pont de matière sécable étant cassé pour retirer ladite partie de protection et ledit corps interne dudit dispositif d'injection.

Avantageusement, la force nécessaire pour retirer ladite partie de fixation dudit dispositif d'injection est supérieure à la force nécessaire pour casser ledit au moins un pont de matière sécable.

Avantageusement, le rabattement est réalisé à chaud, typiquement entre 100°C et 200 °C.

Avantageusement, ledit corps interne, lorsqu'il est soumis à des températures élevées, par exemple une stérilisation à la vapeur à environ 120°C, présente des modifications dimensionnelles inférieures à 2%, avantageusement inférieures à 1%, de préférence inférieures à 0,5%. Avantageusement, ledit dispositif d'injection est une seringue. Avantageusement, ledit dispositif d'injection est un autoinjecteur.

Ces caractéristiques et avantages et d'autres de la présente invention apparaîtront plus clairement à partir de la description détaillée suivante, faite en référence aux dessins joints donnés à titre d'exemples non-limitatifs, sur lesquels :
- les figures 1 et 2 sont des vues schématiques en section transversale d'un dispositif de protection d'aiguille selon une première variante de réalisation avantageuse, respectivement en position de stockage sur un dispositif d'injection et en position retirée, et
- la figure 3 est une vue schématique en section transversale d'une variante de réalisation avantageuse, en position de stockage.

La présente invention va être décrite en référence à plusieurs variantes de réalisation d'un dispositif de protection d'aiguille pour dispositif d'injection. Il est toutefois entendu que la présente invention n'est pas limitée par les modes de réalisation représentés sur les dessins.

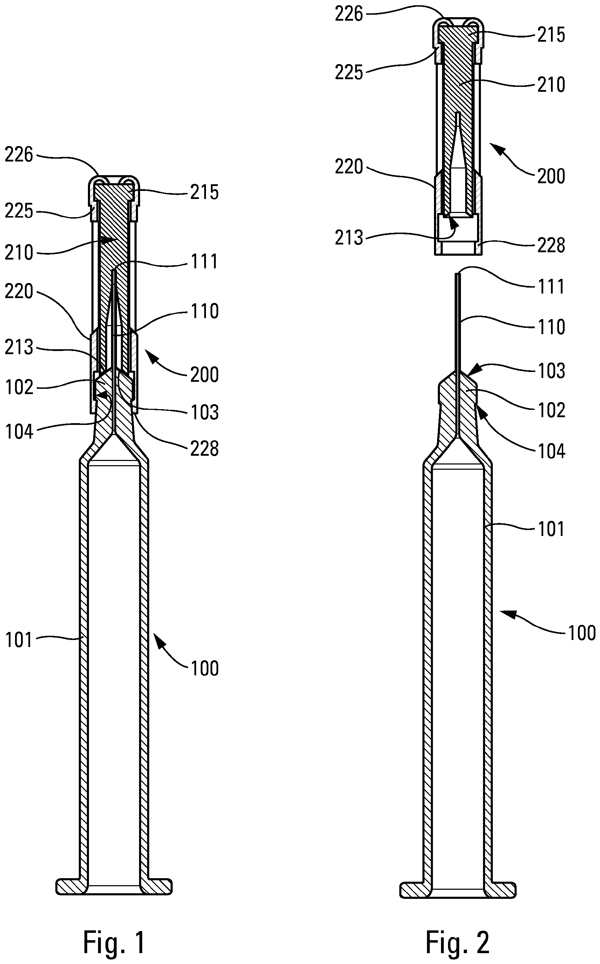

En référence aux figures 1 et 2, il est représenté un dispositif d'injection qui dans cet exemple est une seringue 100 pourvue d'un corps de seringue 101 et d'une aiguille 110 comportant un orifice de distribution 111. La partie d'extrémité axiale du corps de seringue qui fixe l'aiguille 110 comporte une projection d'extrémité axiale 102, généralement appelée boule de la seringue, définie entre la surface d'extrémité axiale 103 du corps de seringue et une projection radiale 104 prévue pour recevoir et fixer un dispositif de protection d'aiguille 200, qui sera décrit ci-après. L'aiguille a typiquement une longueur de 12,7 mm (1/2 pouce), 15,9 mm (5/8 pouce) ou 25,4 mm (1 pouce). D'autres dimensions sont aussi envisageables.

Un dispositif de protection d'aiguille 200 est prévu pour protéger et maintenir étanche ladite aiguille jusqu'à l'utilisation du dispositif d'injection. Le dispositif de protection d'aiguille 200 est fixé sur ledit dispositif d'injection 100 dans une position de stockage, et il est amovible, c'est-à-dire qu'il peut être retiré dudit dispositif d'injection au moment de l'utilisation dudit dispositif d'injection. Le dispositif de protection d'aiguille 200 comporte un corps interne 210 et un corps externe 220.

Le corps interne 210 est réalisé en matériau sensiblement souple ou déformable. Le corps interne 210 comporte une partie pleine qui, en position de stockage du dispositif de protection d'aiguille, reçoit l'orifice de distribution 111 de l'aiguille 110. L'extrémité axiale de l'aiguille 110 est donc enfoncée dans ledit corps interne 210 dans ladite position de stockage. Le corps interne 210 comporte aussi une projection radiale 215 formée à l'extrémité axiale distale dudit corps interne par rapport audit dispositif d'injection. De l'autre coté, à l'autre extrémité axiale, le corps interne 210 forme un manchon creux, et comporte un bord d'extrémité axial 213 proximal par rapport au dispositif d'injection dont la forme est complémentaire de la surface d'extrémité axiale 103 de la boule de la seringue 102.

Le corps externe 220 est réalisé, de préférence de manière monobloc, en un matériau sensiblement rigide, tel que par exemple le polypropylène (PP), le polystyrène (PS), le polyoxyméthylène (POM) ou le polybutylène téréphtalate (PBT). D'autres matériaux sont aussi envisageables. Le corps externe 220 comporte une partie de fixation 228 qui est adaptée à coopérer avec le corps de seringue 101, notamment avec la boule de la seringue 102, et plus particulièrement avec l'épaulement radial 104, pour fixer, notamment par encliquetage, ledit dispositif de protection d'aiguille 200 sur ledit dispositif d'injection 100. Le corps externe 220 comporte de l'autre coté, à savoir du coté distal par rapport au dispositif d'injection, un épaulement radial 225 et un bord d'extrémité axial 226 qui définit une ouverture axiale supérieure. Ledit bord d'extrémité axial est rabattable vers l'intérieur, comme cela sera expliqué ultérieurement.

Le dispositif de protection d'aiguille 200 est avantageusement réalisé par moulage des corps interne 210 et externe 220, puis assemblage du corps interne dans le corps externe. De préférence, le corps interne est inséré dans le corps externe à travers l'ouverture supérieure, jusqu'à ce que la projection radiale 215 du corps interne 210 vienne en appui sur l'épaulement radial 225 du corps externe 220. Le bord d'extrémité axial 226 du corps externe 220 est alors rabattu pour maintenir fixement le corps interne 210 dans le corps externe 220. Ce rabattement peut être réalisé avant ou après assemblage du dispositif de protection d'aiguille 200 sur le dispositif d'injection 100. Le rabattement du bord d'extrémité axial 226 est de préférence réalisé à chaud, typiquement entre à 100°C et 200°C en fonction du matériau du corps externe.

En position de stockage, l'étanchéité avec l'aiguille 110 est réalisée par l'enfoncement de l'orifice de distribution 111 de l'aiguille 110 dans le matériau souple ou déformable du corps interne 210.

Selon l'invention, l'étanchéité avec le dispositif d'injection est réalisée exclusivement par contact entre le bord d'extrémité axial 213 du corps interne 210 et la surface d'extrémité axiale 103 de la boule de la seringue 102. Ledit corps interne 210 est réalisé en un matériau présentant les propriétés suivantes : une dureté supérieure à 55 Shore A, avantageusement supérieure à 60 Shore A, une élasticité supérieure à 8 MPa, avantageusement supérieure à 10 MPa, une déformation rémanente à la compression inférieure à 30%, avantageusement inférieure à 25%, une densité supérieure à 1, avantageusement supérieure à 1,3. Plus particulièrement, ledit corps interne est réalisé en caoutchouc.

Pour obtenir une bonne étanchéité, il est nécessaire de comprimer le corps interne 210 sur la boule 102 de la seringue. Garantir une étanchéité à ce niveau est critique du fait de la faible surface disponible : environ 12mm², comparativement aux dispositifs de protection qui font l'étanchéité au moins en partie sur le coté latéral de la boule de la seringue, et qui disposent d'au moins 40 mm².

Le caoutchouc s'avère bien meilleur qu'un TPE (thermoplastique élastomère) pour réaliser le corps interne selon l'invention. En effet, un caoutchouc permet de transmettre une force plus importante au niveau de la surface d'étanchéité car il combine une dureté et une élasticité plus importantes comparativement à un TPE.

Pour le caoutchouc, la dureté est supérieure à 55 Shore A, généralement même supérieure à 60 Shore A et l'élasticité est supérieure à 8 MPa, généralement même supérieure à 10 MPa. Ceci doit être comparé à des duretés de TPE n'allant pas au dessus de 55 Shore A et des élasticités oscillantes entre 4 et 12 MPa. Il est à noter que pour les TPE, jamais une dureté importante n'est associée à une élasticité importante. En effet, la dureté du TPE étant aussi fonction de l'élasticité de celui-ci, plus il aura une dureté importante moins bonne sera son élasticité. Ceci n'est pas le cas des caoutchoucs.

Un caoutchouc pourra dans le temps garantir une compression homogène déterminée car sa DRC (déformation rémanente à la compression) est moins importante que celle d'un TPE. Le caoutchouc à typiquement une DRC inférieure à 30%, généralement même inférieure à 25%, comparativement aux TPE qui ont des DRC pouvant aller à des valeurs supérieures à 40%.

Un caoutchouc a aussi une stabilité dimensionnelle qui lui permet de subir différents traitements, comme la stérilisation vapeur. Ceci se traduit par une différence notable avec un TPE, qui peut subir un retrait, c'est-à-dire une diminution dimensionnelle. En effet, à température élevée, par exemple environ 120°C pendant la stérilisation vapeur, le TPE peut avoir un retrait pouvant aller jusqu'à 3%. Ceci se traduit alors par une réduction de sa longueur totale et une perte d'étanchéité. Le caoutchouc ne présente pas cette variation dimensionnelle, notamment lors de la stérilisation vapeur. En particulier, un caoutchouc, lorsqu'il est soumis à des températures élevées, par exemple une stérilisation à la vapeur à environ 120°C, présente des modifications dimensionnelles inférieures à 2%, avantageusement inférieures à 1%, de préférence inférieures à 0,5%.

Les matériaux suivants peuvent notamment être utilisés dans le cadre de la présente invention : mélanges d'élastomères caoutchouc incluant dans leur composition au moins un des élastomères caoutchouc suivants : les copolymères butadiène-styrène (SBR), les polybutadiènes (BR), les polyisoprènes naturels (IR) ou de synthèse, les copolymères à base d'isobutylène et d'isoprène, halogénés ou non (IIR, CIIR, BIIR), les copolymères ou ter-polymères à base d'éthylène et de propylène (EPM, EPDM) et les copolymères butadiènes-acrylonitrile (NBR)

Ces mélanges de caoutchouc sont composés de polymères de différentes natures, et ils peuvent comporter des charges minérales, de type noir de carbone, silices, kaolins, talc, etc.

Ces mélanges de caoutchouc peuvent également comporter différents types d'agents de vulcanisations, de plastifiants, de colorants ou d'agents de protections.

Le tableau suivant compare les propriétés d'un caoutchouc à base de polyisoprène avec différents TPE, respectivement à base de SEBS (polystyrène-b-poly(éthylène-butylène)-b-polystyrène) et d'EPDM (éthylène-propylène-diène monomère).

| | | Caoutchouc à base de polyisoprène | TPE à base de SEBS | TPE à base d'EPDM | Méthode |
|---|---|---|---|---|---|
| Densité | - | 1,35 | 0,92 | 0,93 | ASTM D 792 |
| Dureté | Shore A | 61 | 51 | 55 | ASTM D 2240 |
| Perméabilité vapeur | g/m².j | 4,98 | 2,88 | 3,28 | ASTM F 1249 eau 38°C, 90% RH |
| Elasticité | MPa | 11,1 | 12 | 4,6 | ASTM D 412 |
| Elongation | % | 500 | 800 | 480 | ASTM D 412 |
| DRC | % | 23 | 42 | 27 | ASTM D 395 (B- 22 h - 70°C) |

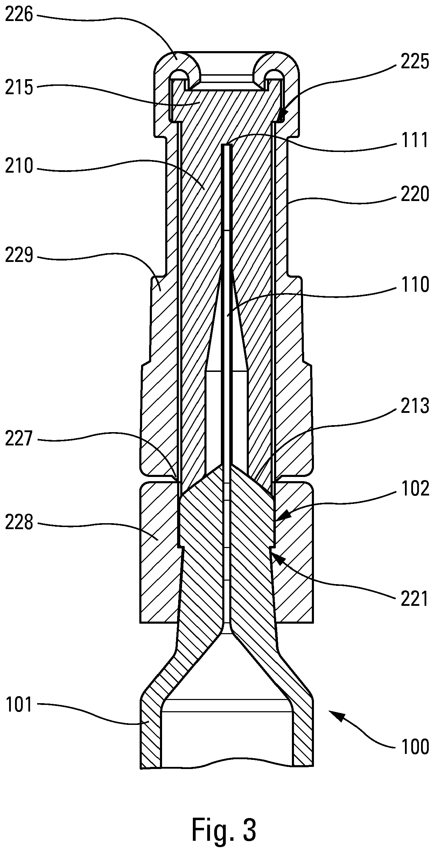

La figure 3 montre une variante de réalisation avantageuse incorporant un témoin de premier usage.

Dans cette variante, le corps externe 220 comporte une partie de protection 229 qui est reliée à la partie de fixation 228 par au moins un pont de matière sécable 227. Ainsi, le corps externe 220 est réalisé d'une seule pièce monobloc, et lors du retrait du dispositif de protection d'aiguille, ledit au moins un pont de matière sécable 227 est cassé, ce qui forme un témoin de premier usage. Pour pouvoir retirer le dispositif de protection d'aiguille, il faut casser le ou les pont(s) de matière sécable(s), et une fois cassés, il n'est plus possible de les remettre à l'état initial. L'utilisateur qui voit les ponts de matières intacts sait donc que le dispositif de protection d'aiguille n'a pas été retiré. Si par contre les ponts de matières sont cassés, il sait qu'il y a un risque de perte d'étanchéité et donc de contamination. Bien entendu, la force nécessaire pour démonter la partie de fixation 228 du dispositif d'injection 100 doit être supérieure à la force nécessaire pour casser le ou les pont(s) de matière. Ainsi, on s'assure que ce seront toujours d'abord les ponts de matière qui casseront, et il ne sera alors pas possible de retirer le dispositif de protection d'aiguille sans casser lesdits ponts de matière.

La partie de fixation 228 est fixée sur le dispositif d'injection, notamment encliquetée sur la boule de la seringue, comme décrit précédemment. La partie de protection 229 est fixée audit corps interne 210, notamment par coincement de la projection radiale 215 du corps interne 210 entre l'épaulement radial 225 et le bord d'extrémité axial rabattu 226.

Dans la variante de réalisation de la figure 3, les ponts de matière 227 sont cassés en tirant axialement sur la partie de protection 229. Lorsque la force est suffisante, les ponts de matière 227 se cassent, et la partie de protection ensemble avec le corps interne 210 peuvent être retirés du dispositif d'injection 100, alors que la partie de fixation 228 reste fixée à la boule de la seringue.

Bien entendu, l'invention n'est pas limitée aux exemples de réalisation représentés sur les dessins, et la portée de l'invention est au contraire définie par les revendications annexées.

## Revendications

1. Dispositif d'injection de produit fluide (100) comportant un corps de seringue (101) et une aiguille (110) fixée dans une projection d'extrémité axiale (102) du corps de seringue, ladite projection d'extrémité (102) comportant une surface d'extrémité axiale (103) et un épaulement radial (104), ledit dispositif d'injection comportant un dispositif de protection d'aiguille (200), ledit dispositif de protection étant, dans une position de stockage, fixé sur ledit dispositif d'injection, ledit dispositif de protection étant amovible dudit dispositif d'injection, ledit dispositif de protection comportant un corps interne (210) en matériau sensiblement souple et/ou déformable et un corps externe (220) en matériau sensiblement rigide, ledit corps interne (210), en position de stockage, obturant de manière étanche l'orifice de distribution (111) de ladite aiguille (110) et coopérant de manière étanche avec ledit dispositif d'injection (100), ledit corps externe (220) comportant une partie de fixation (228) qui, en position de stockage, coopère par encliquetage avec ledit épaulement radial (104) de ladite projection d'extrémité (102) pour fixer ledit dispositif de protection (200) sur ledit dispositif d'injection (100), ledit corps interne (210) comporte un bord d'extrémité axial proximal (213) adapté, en position de stockage, à coopérer de manière étanche avec le corps de seringue (101) uniquement avec ladite surface d'extrémité axiale (103), et ledit corps interne (210) étant réalisé en un matériau présentant les propriétés suivantes : une dureté supérieure à 55 Shore A, notamment supérieure à 60 Shore A, une élasticité supérieure à 8 MPa, notamment supérieure à 10 MPa, une déformation rémanente à la compression inférieure à 30%, notamment inférieure à 25%, une densité supérieure à 1, notamment supérieure à 1,3.

2. Dispositif selon la revendication 1, dans lequel ledit corps interne (210) est réalisé en caoutchouc.

3. Dispositif selon la revendication 1 ou 2, dans lequel ledit corps interne (210) comporte une projection radiale (215) et ledit corps externe (220) comporte un épaulement radial (225) et un bord d'extrémité radial (226) définissant une ouverture axiale supérieure, ledit corps interne (210) étant inséré dans ledit corps externe (220) à travers ladite ouverture axiale supérieure, avec ladite projection radiale (215) en butée sur ledit épaulement radial (225), ledit bord d'extrémité axial (226) dudit corps externe (220) étant rabattu sur ledit corps interne (210) pour fixer ledit corps interne (210) dans ledit corps externe (220).

4. Dispositif selon l'une des revendications précédentes, dans lequel ledit corps externe (220) comporte une partie de protection (229) fixée audit corps interne (210), ladite partie de protection (229) reliée à ladite partie de fixation (228) par au moins un pont de matière sécable (227), ledit au moins un pont de matière sécable (227) étant cassé pour retirer ladite partie de protection (229) et ledit corps interne (210) dudit dispositif d'injection (100).

5. Dispositif selon la revendication 4, dans lequel la force nécessaire pour retirer ladite partie de fixation (228) dudit dispositif d'injection (100) est supérieure à la force nécessaire pour casser ledit au moins un pont de matière sécable (227).

6. Dispositif selon l'une quelconque des revendications précédentes, dans lequel le rabattement est réalisé à chaud, typiquement entre 100°C et 200°C.

7. Dispositif selon l'une quelconque des revendications précédentes, dans lequel ledit corps interne (210), lorsqu'il est soumis à des températures élevées, par exemple une stérilisation à la vapeur à environ 120°C, présente des modifications dimensionnelles inférieures à 2%, avantageusement inférieures à 1%, de préférence inférieures à 0,5%.

8. Dispositif selon l'une quelconque des revendications précédentes, dans lequel ledit dispositif d'injection est une seringue.

9. Dispositif selon l'une quelconque des revendications précédentes, dans lequel ledit dispositif d'injection est un autoinjecteur.

## Patentansprüche

1. Vorrichtung zur Injektion eines flüssigen Produkts (100), umfassend einen Spritzenkörper (101) und eine Nadel (110), die an einem axialen Endvorsprung (102) des Spritzenkörpers angebracht ist, wobei der Endvorsprung (102) eine axiale Endoberfläche (103) und einen radialen Vorsprung (104) umfasst, die Injektionsvorrichtung eine Nadelschutzvorrichtung (200) umfasst, wobei diese Schutzvorrichtung in einer Aufbewahrungsstellung an der Injektionsvorrichtung befestigt ist, wobei die Schutzvorrichtung von der Injektionsvorrichtung abnehmbar ist, wobei die Schutzvorrichtung einen Innenkörper (210) aus einem im Wesentlichen weichen und/oder verformbaren Material sowie einen Außenkörper (220) aus einem im Wesentlichen steifen Material umfasst, wobei der Innenkörper (210) in einer Aufbewahrungsstellung die Abgabeöffnung (111) der Nadel (110) dicht verschließt und mit der Injektionsvorrichtung (100) abdichtend zusammenwirkt, wobei der Außenkörper (220) einen Befestigungsabschnitt (228) umfasst, der in der Verwahrungsstellung durch Verrastung am radialen Vorsprung (104) des Endvorsprungs (102) die Schutzvorrichtung (200) an der Injektionsvorrichtung (100) festlegt,
wobei der Innenkörper (210) einen axialen proximalen Endrand (213) umfasst, der derart ausgelegt ist, dass er in Verwahrungsstellung ausschließlich über die Fläche des axialen Endrands (103) dichtend mit dem Spritzenkörper (101) zusammenwirkt, und
der Innenkörper (210) aus einem Material mit folgenden Eigenschaften ausgeführt ist: einer Shore-A-Härte von über 55, insbesondere über 60, einer Elastizität von über 8 MPa, insbesondere über 10 MPa, einer bleibenden Druckverformung von unter 30%, insbesondere unter 25%, einer Dichte von über 1, insbesondere über 1,3.

2. Vorrichtung nach Anspruch 1, bei der der Innenkörper (210) aus Kautschuk gebildet ist.

3. Vorrichtung nach Anspruch 1 oder 2, bei der der Innenkörper (210) einen radialen Vorsprung (215) und der Außenkörper (220) einen radialen Absatz (225) und einen radialen Endrand (226) umfasst, die eine obere axiale Öffnung definieren, wobei der Innenkörper (210) über die obere axiale Öffnung in den Außenkörper (220) eingesetzt wird, wobei der radiale Vorsprung (215) an dem radialen Absatz (225) anliegt, wobei der axiale Endrand (226) des Außenkörpers (220) über den Innenkörper (210) zurückgebogen ist, um den Innenkörper (210) im Außenkörper (220) zu fixieren.

4. Vorrichtung nach einem der vorhergehenden Ansprüche, bei der der Außenkörper (220) einen Schutzabschnitt (229) umfasst, der am Innenkörper (210) befestigt ist, wobei der Schutzabschnitt (229) über mindestens einen Steg (227) aus zerbrechlichem Material mit dem Befestigungsabschnitt (228) verbunden ist, wobei der mindestens eine Steg (227) aus zerbrechlichem Material zum Entfernen des Schutzabschnitts (229) mit Innenkörper (210) von der Injektionsvorrichtung (100) zerbrochen wird.

5. Vorrichtung nach Anspruch 4, bei der die zum Entfernen des Befestigungsabschnitts (228) von der Injektionsvorrichtung (100) aufzubringende Kraft größer ist als die zum Zerbrechen des mindestens einen Stegs (227) aus zerbrechlichem Material erforderliche Kraft.

6. Vorrichtung nach einem der vorhergehenden Ansprüche, bei der das Zurückbiegen unter Hitzeeinwirkung erfolgt, üblicherweise bei einer Temperatur zwischen 100°C und 200°C.

7. Vorrichtung nach einem der vorhergehenden Ansprüche, bei der sich die Abmessungen des Innenkörpers (210) unter erhöhten Temperaturen, beispielsweise einer Dampfsterilisation bei ca. 120°C, um weniger als 2%, vorteilhafterweise weniger als 1%, und vorzugsweise weniger als 0,5% verändern.

8. Vorrichtung nach einem der vorhergehenden Ansprüche, bei der die Injektionsvorrichtung eine Spritze ist.

9. Vorrichtung nach einem der vorhergehenden Ansprüche, bei der die Injektionsvorrichtung ein Autoinjektionsgerät ist.

## Claims

1. An injection device of fluid product (100) comprising a syringe body (101) and a needle (110) fixed in an axial end projection (102) of the syringe body, said end projection (102) comprising an axial end surface (103) and a radial shoulder (104), said injection device comprising a needle protection device (200), said protection device being, in a storage position, fixed on said injection device, said protection device being removable from said injection device, said protection device comprising an inner body (210) made of substantially supple and/or deformable material and an outer body (220) made of substantially rigid material, said inner body (210), in a storage position, sealingly closing the distribution orifice (111) of said needle (110) and sealingly cooperating with said injection device (100), said outer body (220) comprising a fixing part (228) which, in a storage position, cooperates by snap fastening with said radial shoulder (104) of said end projection (102) to fix said protection device (200) onto said injection device (100), said inner body (210) comprises a proximal axial end edge (213) adapted, in a storage position, to sealingly cooperate with the syringe body (101) only with said axial end surface (103), and said inner body (210) being made of material having the following properties: a hardness greater than 55 Shore A, in particular greater than 60 Shore A, an elasticity greater than 8 MPa, in particular greater than 10 MPa, a compression set less than 30%, in particular less than 25%, a density greater than 1, in particular greater than 1.3.

2. The device according to claim 1, wherein said inner body (210) is made of rubber.

3. The device according to claim 1 or 2, wherein said inner body (210) comprises a radial projection (215) and said outer body (220) comprises a radial shoulder (225) and a radial end edge (226) defining an upper axial opening, said inner body (210) being inserted into said outer body (220) through said upper axial opening, with said radial projection (215) stopped on said radial shoulder (225), said axial end edge (226) of said outer body (220) being folded back on said inner body (210) to fix said inner body (210) in said outer body (220).

4. The device according to any one of the preceding claims, wherein said outer body (220) comprises a protection part (229) fixed to said inner body (210), said protection part (229) connected to said fixing part (228) by at least one breakable material bridge (227), said at least one breakable material bridge (227) being broken to remove said protection part (229) and said inner body (210) from said injection device (100).

5. The device according to claim 4, wherein the force necessary to remove said fixing part (228) from said injection device (100) is greater than the force necessary to break said at least one breakable material bridge (227).

6. The device according to any one of the preceding claims, wherein folding back is carried out under heat, typically between 100°C and 200°C.

7. The device according to any one of the preceding claims, wherein said inner body (210), when subjected to high temperatures, steam sterilisation at around 120°C for example, has dimensional modifications less than 2%, advantageously less than 1%, preferably less than 0.5%.

8. The device according to any one of the preceding claims, wherein said injection device is a syringe.

9. The device according to any one of the preceding cl+aims, wherein said injection device is an auto-injector.
